(19) Europäisches Patentamt
European Patent Office
Office européen des brevets

(11) EP 3 824 888 A1

(12) EUROPEAN PATENT APPLICATION
published in accordance with Art. 153(4) EPC

(43) Date of publication:
**26.05.2021 Bulletin 2021/21**

(21) Application number: **19837086.8**

(22) Date of filing: **29.05.2019**

(51) Int Cl.:
***A61K 31/4436** (2006.01)* ***A61K 9/20** (2006.01)*
***A61K 9/48** (2006.01)* ***A61P 31/04** (2006.01)*

(86) International application number:
**PCT/KR2019/006432**

(87) International publication number:
**WO 2020/017756 (23.01.2020 Gazette 2020/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **20.07.2018 KR 20180084538**

(71) Applicant: **Crystalgenomics, Inc.**
**Seongnam-si, Gyeonggi-do 13488 (KR)**

(72) Inventors:
- **CHO, Jae Pyoung**
  **Gunpo-si, Gyeonggi-do 15822 (KR)**
- **CHO, Joong Myung**
  **Seoul 05553 (KR)**

(74) Representative: **Schiweck Weinzierl Koch Patentanwälte Partnerschaft mbB**
**Ganghoferstraße 68 B**
**80339 München (DE)**

# (54) ORALLY ADMINISTERED PHARMACEUTICAL COMPOSITION COMPRISING FAB I INHIBITORS AND METHOD FOR PREPARING SAME

(57) The present invention relates to an orally administered pharmaceutical composition comprising Fab I inhibitors, and to a method for preparing same. The present invention may be applied effectively to bacterial infections resistant to antibiotics and the like. More specifically, the present invention can more rapidly initiate therapeutic effects by improving dissolution and elution rates. Furthermore, the present invention can improve the mixing and content uniformity in preparations by regulating the particle size.

[Fig. 1]

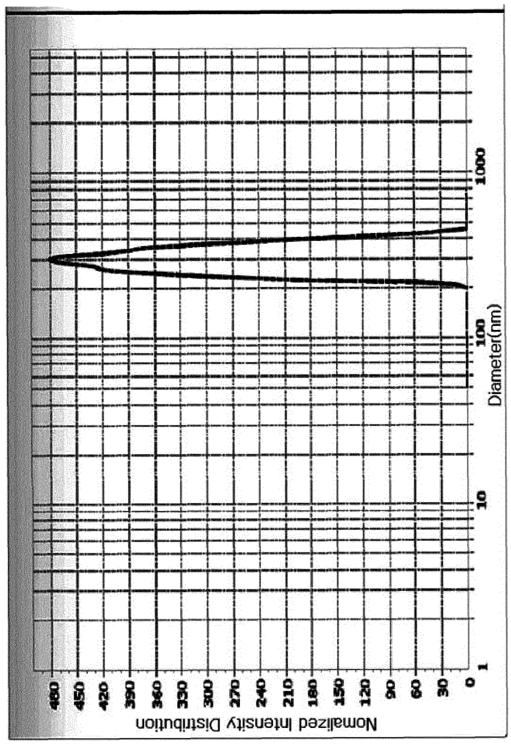


EP 3 824 888 A1

## Description

BACKGROUND OF THE INVENTION

1. Field of the Invention

The present invention relates to an orally administered pharmaceutical composition comprising Fab I inhibitors, and to a method for preparing same.

2. Description of the Related Art

Infectious diseases caused by bacteria are diseases that have plagued humans for as long as human history and have been a great influence on human history, such as the Black Death. In order to overcome these threats from bacteria, humans have made ceaseless efforts, which has led to the rapid development of medicals and medicines. The development of the modern concept of antibiotics began in 1928 with penicillin, first discovered by Alexander Fleming. Since then, the development of antibiotics to treat bacterial infections has made a leap forward. However, the resistance of the bacteria itself to antibiotics began to be known, and the use of antibiotics was restricted.

Since then, as the development of novel antibiotics and the continuous appearance of resistant bacteria against them have been repeated, the development of the novel antibiotics has become a necessary task for the treatment of bacterial infections. In addition, research strategies are also being changed to overcome resistant bacteria. Interest is focused on the development of antibiotics having a new mechanism of action because the resistance that has already been expressed cannot be overcome even though new antibiotics with better efficacy is developed using the previously established bacterial inhibitory mechanism of action. In addition, large pharmaceutical companies such as Bayer, Bristol-Myers Squibb, Merck, Glaxo Smith Kline and Astrazeneca around the world are making great efforts to develop a new concept of antibiotics that can overcome resistance through a completely different mechanism of action from conventional antibiotics. Among these resistant strains, one of the most difficult strains to be treated is MRSA (Methicillin-Resistant Staphylococcus Aureus). MRSA is a staphylococcus aureus that is resistant to methicillin, a penicillin antibiotic. It is not only resistant to methicillin, but has strong resistance to most antibiotics, so it is a pathogen that can be treated only with very limited antibiotics. The outbreak of MRSA infection is on the rise worldwide. The reason this strain is attracting attention is not only because it is resistant to existing antibiotics, but also it is the most frequent causative organism among pathogens that induce in-hospital infection and can be fatal to patients with weak immunity or to the old and infirm. In recent years, not only healthcare-acquired MRSA infections but also community-acquired MRSA infections are increasing significantly, indicating exposure to MRSA occurs easily in everyday life. Vancomycin has been used for its treatment. However, strains resistant to vancomycin have been reported. Other therapeutic agents include Linezolid and Daptomycin, but the selection of antibiotics for therapeutic purpose is very limited. Therefore, there is an urgent need to develop novel antibiotics that can be used for antibiotic resistant bacterial infections.

SUMMARY OF THE INVENTION

In order to solve the above problems, the present invention provides a pharmaceutical composition for oral administration comprising Fab I inhibitors and salts thereof as an active ingredient.

In addition, it provides a method for preparing a pharmaceutical composition for oral administration comprising Fab I inhibitors and salts thereof as an active ingredient.

The present invention provides a pharmaceutical composition for oral administration comprising Fab I inhibitors, including 1-(3-amino-2-methylbenzyl)-4-(2-thiophen-2-yl-ethoxy)-1H-pyridin-2-one, a salt thereof, or a combination thereof.

According to one embodiment, the composition may have a particle size distribution $D_{90}$ of 0.1 to 500 $\mu$m.

According to one embodiment, the composition may be provided in the form of a tablet or capsule.

According to one embodiment, the 1-(3-amino-2-methylbenzyl)-4-(2-thiophen-2-yl-ethoxy)-1H-pyridin-2-one, a salt thereof or a combination thereof may be present in an amount of 10 to 60% by weight based on the total weight of the composition.

According to other aspect of the present invention, there is provides a method for preparing a pharmaceutical composition for oral administration comprising Fab I inhibitors, the method comprising:

adding 4-benzyloxy-1H-pyridone, 2-methyl-3-nitro-benzylchloride and potassium tert-butoxide to dimethylformamide, and mixing them to react with heating;
adding purified water and drying with heating to obtain a dried product;
dissolving the dried product in an organic solvent and adding purified water for layer separation;

recovering the organic layer to filter and concentrate it to obtain concentrates;
re-concentrating the concentrates and adding hexane to obtain precipitates;
dissolving the resulting precipitates, and cooling, filtering and drying them to obtain a dried product; and
dissolving the resulting dried product in an organic solvent, adding iron chloride hexahydrate, activated carbon and hydrazine monohydrate thereto, cooling and filtrating them to obtain resulting precipitates and then drying and pulverizing it.

According to one embodiment, the method may further comprise adding an acidic substance.
According to one embodiment, the acidic substance may include hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, oxalic acid, fumaric acid, malonic acid, maleic acid, methanesulfonic acid, benzenesulfonic acid, p-toluenesulfonic acid, naphthalenesulfonic acid, EDTA, and combinations thereof.
According to one embodiment, the precipitates may be formulated into a tablet containing nanoparticles or a capsule containing solid dispersion particles.
According to one embodiment, the method may comprise filling into the capsule the resulting precipitates in the form of solid dispersion particles comprising hydrophilic polymers.
According to one embodiment, the composition of the present invention may be used for the treatment of bacterial infections.
Other specifics of the embodiments of the present invention are included in the detailed description below.

## EFFECT OF THE INVENTION

The pharmaceutical composition for oral administration comprising Fab I inhibitors according to the present invention can be applied effectively to bacterial infections resistant to antibiotics and the like. More specifically, the present invention can more rapidly initiate therapeutic effects by improving dissolution and elution rates. Furthermore, the present invention can improve the mixing and content uniformity in preparations by regulating the particle size.

## BRIEF DESCRIPTION OF THE DRAWINGS

Fig. 1 is a graph showing the results of a particle size measurement of nanoparticles.
Figs. 2 and 3 are graphs showing dissolution patterns in distilled water of each of Preparation Examples and Examples.
Fig. 4 is a graph showing a dissolution pattern for pH 1.2 aqueous solution of each Example.
Fig. 5 is a graph showing antibacterial effect depending on T/MIC values.
Fig. 6 is a graph showing concentrations of drugs in serum over time.

## DETAILED DESCRIPTION OF THE INVENTION

Since various modifications and variations can be made in the present invention, particular embodiments are illustrated in the drawings and will be described in detail in the detailed description. It should be understood, however, that the invention is not intended to be limited to the particular embodiments, but includes all modifications, equivalents, and alternatives falling within the spirit and scope of the invention. In the following description of the present invention, detailed description of known functions will be omitted if it is determined that it may obscure the gist of the present invention.
Hereinafter, the pharmaceutical composition according to an embodiment of the present invention will be described in more detail.
The term "pharmaceutical composition" as used herein may be described interchangeably with "pharmacological composition" and "pharmaceutically acceptable composition" and refers to any composition which can be a relatively non-toxic to a subject to be administered and have harmless effective action. In addition, it may refer to any organic or inorganic compound formulation in that side effects resulting from the composition do not impair the efficacy of the drug, and that does not cause serious irritation to a subject to be administered by the compound and does not impair the biological activities and properties of the compound.
As used herein, the term "subject to be administered" may be used interchangeably with "individual to be administered" and "organism to be administered" and may refer to any animals including humans in which acute or chronic pain is caused or may be caused.
In addition, the term "bacterial infection" may be used interchangeably with "bacteria-related disease" and refers

to a disorder or disease caused by bacterial infection. The disorder or disease may include, for example, urinary tract, respiratory or skin tissue infection, sepsis, but is not limited thereto.

The present invention provides a pharmaceutical composition for oral administration comprising Fab I inhibitors. Specifically, in the present invention, selective Fab I inhibitors may include 1-(3-amino-2-methylbenzyl)-4-(2-thiophen-2-yl-ethoxy)-1H-pyridin-2-one, a salt thereof, or a combination thereof. 1-(3-amino-2-methylbenzyl)-4-(2-thiophen-2-yl-ethoxy)-1H-pyridin-2-one is represented by the following formula 1.

[Formula 1]

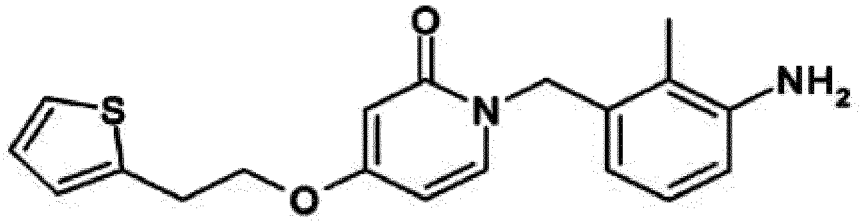

Chemical name: 1-(3-amino-2-methylbenzyl)-4-(2-thiophen-2-yl-ethoxy)-1H-pyridin-2-one

The selective Fab I inhibitors, for example, having the structure of formula 1 are compounds that have a completely different mechanism of action from the existing antibiotics, such as beta-lactam antibiotics (penicillin, cephalosporin, etc.), glycopeptides (vancomycin, etc.), tetracyclines, aminoglycosides, glycylclines, macrolides, chloramphenicol, quinolones, sulfonamides, and oxazolines, which exhibit antibiotic efficacy by inhibiting the action of the enzyme Fab I essential for protein synthesis in bacteria.

Fatty acids, which are not only an energy source for living organisms but also a major component of cell membranes, play an essential role in maintaining life phenomena. Therefore, biosynthesis processes of the fatty acids in cells are essential biochemical processes that exist in all living cells. Genes involved in these processes are one of essential genes in from bacteria to humans.

Fab I, which is an enoyl-ACP reductase in the final step of the cycle, among the four enzymes involved in bacterial fatty acid biosynthesis, has been reported to play a role in converting enoyl-ACP to the corresponding acyl-ACP through a 1,4-reduction reaction ((Payne et al., Drug Discovery Today 6, 2001, 537-544). Fab I is the most important protein in fatty acid synthesis and involved in the reaction that determines the rate of the overall synthesis process. However, in mammals such as humans, unlike bacteria, a huge group of enzymes called fatty acid synthases are used for the synthesis of such fatty acids. Moreover, their structures are completely different from the proteins in the bacterial fatty acid synthesis pathway. Therefore, since selective Fab I inhibitors have little toxicity and are inhibitors against a novel target protein that has not been targeted with any antibiotic until now, the development of drugs that act on this target protein can improve a treatment success rate against bacteria having drug resistance, especially multidrug resistance.

According to one embodiment, the compound of formula 1 may be provided in the form of an amorphous form, a crystalline form, or a mixture thereof, and for example, the compound of formula 1, a salt thereof or a combination thereof may be included in an amount of 10 to 60% by weight, for example 20 to 40% by weight, for example 10 to 30% by weight, for example 30 to 60% by weight.

According to other aspect of the present invention, there is provides a method for preparing a pharmaceutical composition for oral administration comprising Fab I inhibitors, the method comprising:

adding 4-benzyloxy-1H-pyridone, 2-methyl-3-nitro-benzylchloride and potassium tert-butoxide to dimethylformamide, and mixing them to react with heating;
adding purified water and drying with heating to obtain a dried product;
dissolving the dried product in an organic solvent and adding purified water for layer separation;
recovering the organic layer to filter and concentrate it to obtain concentrates;
re-concentrating the concentrates and adding hexane to obtain precipitates;
dissolving the resulting precipitates, and cooling, filtering and drying them to obtain a dried product; and
dissolving the resulting dried product in an organic solvent, adding iron chloride hexahydrate, activated carbon and hydrazine monohydrate thereto, cooling and filtrating them to obtain resulting precipitates and then drying and pulverizing it.

According to one embodiment, the pharmaceutical acceptable salt of the compound of formula 1 may be contained in the pharmaceutical composition for oral administration. The pharmaceutical acceptable salt may be an acid

addition salt formed using an acid. Examples of the acid include, but are not limited to, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, oxalic acid, fumaric acid, malonic acid, maleic acid, methanesulfonic acid, benzenesulfonic acid, p-toluenesulfonic acid, naphthalenesulfonic acid and EDTA.

According to one embodiment, the present invention can improve the dissolution rate and uniformity in preparations by regulating the particle size of the compound of formula I or a salt thereof. Specifically, the particle size distribution $D_{90}$ of the compound of formula I or a salt thereof may be 0.1 to 500 μm, for example 0.1 to 300 μm, for example 0.1 to 50 μm, for example 0.1 to 25 μm.

According to one embodiment, the pulverization of the compound of formula I or a salt thereof may be pulverized by a wet method or a dry method, but the present invention is not particularly limited thereto, and may be appropriately selected if it is a general pulverization method. For example, an air jet mill, a fluid energy mill, a micron mill, and the like, may be used, the present invention is not limited thereto.

According to one embodiment, the present invention may be provided in an oral dosage form and may be formulated in a solid or liquid form. Specifically, the composition according to present invention may be provided in liquid or solid form and may be provided in any convenient form, such as in the form of tablets, pellets, granules, capsules, suspensions, emulsions or powders, which are suitable for reconstitution with water or other suitable liquid media.

According to one embodiment, the composition of the present invention may be provided in the form of a capsule or tablet. The capsule or tablet may be prepared by solubilizing or mixing them in non-toxic pharmaceutically acceptable excipients suitable for oral administration.

According to a specific embodiment, the composition according to the present invention may be provided in a finished form by additionally adding an excipient and a solubilizing agent to completely dissolve it, spray-drying to prepare powders, and then filling the resulting powders into a hard capsule.

For example, excipients include water-soluble polymers, for example dextrin, polydextrin, dextran, pectin and pectin derivatives, alginate, starch, hydroxypropyl methylcellulose, hydroxypropylcellulose, hydroxymethylcellulose, hydroxylethylcellulose, methyl cellulose, sodium carboxymethyl cellulose, hydroxypropyl methylcellulose acetate succinate, hydroxylethylmethyl cellulose, guar gum, locust bean gum, tragacantha, carrageenan, acacia gum, arabic gum, gellan gum, xanthan gum, gelatin, casein, polyvinyl alcohol, polyvinyl pyrrolidone, polyvinylacetaldiethylaminoacetate, poly(butyl methacrylate, (2-dimethylaminoethyl)methacrylate, (methyl methacrylate) copolymer, polyethylene glycol, polyethylene oxide, carbomer and the like. More specific examples of excipients include polyvinylpyrrolidone. The excipient may be present in an amount of 10 to 60% by weight, for example 30 to 55% by weight, for example 40 to 50% by weight based on the total weight of the composition.

For example, the solubilizing agent includes, but is not limited to, polyhydric alcohols, surfactants, and the like, for example propylene glycol, polyethylene glycol, dipropyleneglycol, diethylene glycol, diethylene glycol monoethyl ether, glycerol, Tween 80, cremophor, transcutol and the like. More specifically, the solubilizing agent includes Tween 80. The solubilizing agent may be present in an amount of 0.5 to 20% by weight, 1 to 10% by weight, for example 2 to 5% by weight based on the total weight of the composition.

According to one embodiment, the compound of formula 1, a salt thereof or a combination thereof may be present in an amount of 10 to 60% such as 30 to 55% by weight, such as 40 to 50% by weight based on the total weight of the composition.

According to a specific embodiment, the composition according to the present invention may be provided in the form of a tablet by adding excipients, disintegrants, and lubricants to form wet granules and combining, drying, sizing and mixing them.

For example, the solvent that can be used for wet granulation may include at least one selected from the group consisting of water, methanol, ethanol, and dichloromethane. Specifically, it may include ethanol or an aqueous ethanol solution, but is not limited thereto.

For example, the excipient may include microcrystalline cellulose, silicified microcrystalline cellulose, mannitol, lactose, silicon dioxide, and the like, but is not limited thereto.

For example, the disintegrant may include croscarmellosesodium, starch, and the like, but is not limited thereto.

For example, the lubricant may include corn starch, talc, magnesium stearate, calcium stearate, polyethylene glycol, sodium lauryl sulfate, and the like, but is not limited thereto.

According to one embodiment, in the preparation of the tablet form of the present invention, the composition may further comprise a binder, including but not limited thereto, gelatin, starch, glucose, povidone, hydroxypropyl cellulose, hydroxypropylmethyl cellulose and the like, for example.

According to one embodiment, the present invention may provide tablets for oral administration, comprising microcrystalline cellulose, D-mannitol, or the like as an excipient, which may be used alone or in combination of two or more; croscarmellosesodium or the like as a disintegrant; and magnesium stearate or the like as a lubricant.

As a specific example, the tablet according to the present invention comprises 10 to 1000 parts by weight, such as 100 to 300 parts by weight of an excipient, 1 to 30 parts by weight, such as 10 to 20 parts by weight of a disintegrant,

0.1 to 20 parts by weight, such as 5 to 15 parts by weight of a binder and 0.1 to 10 parts by weight, such as 3 to 5 parts by weight of a lubricant, based on 100 parts by weight of the compound of formula 1, a salt thereof, or a combination thereof.

According to one embodiment, a pharmaceutically acceptable solvent may be used to dissolve the compound of formula 1 or a salt thereof, and it may include an aqueous solution containing an acid having pH of 1 to 3, for example, pH 1.2, water, methanol, ethanol, dichloromethane, and the like, but is not limited thereto.

According to one embodiment, the present invention can be used for the treatment of gram-positive bacterial infections such as MRSA (methicillin resistant staphylococcus aureus) or various infectious diseases thereof. Gram-positive bacteria include, for example, *Staphylococcus,* such as *Staphylococcus aureus* and *Staphylococcus epidermidis;* and *Streptococcus,* such as *Streptococcus pneumonia, Streptococcus pyrogenes,* group C/F/G *Streptococci* and viridans group *Streptococci.*

According to one embodiment, the composition of the present invention may further comprise additives which are pharmaceutically acceptable and physiologically suitable.

For example, any one may be used as an additive as long as it is pharmaceutically acceptable and commonly used in each formulation, such as fillers, extenders, binders, disintegrants, glidants, preservatives, buffers, coating agents, sweetening agents, solubilizing agents, suspending agents, coloring agents, water-soluble additives, excipients, carriers, fillers, lubricants, desiccants, etc. For example, the additive may be present in an amount of 5 to 90% by weight, for example, 40 to 90% by weight based on the total weight of the composition.

The pharmacological or pharmaceutical composition according to the present invention may be prepared in any form suitable for application to humans, including infants, children, adults and animals, by standard procedures known to those skilled in the art.

Hereinafter, embodiments of the present invention will be described in detail so that those skilled in the art can easily carry out the present invention. The present invention may, however, be embodied in many different forms and should not be construed as limited to the embodiments set forth herein.

Preparation Example 1: Preparation of compound of formula 1

To prepare the compound of formula 1, 0.9 mol of 4-benzyloxy-1H-pyridone was introduced into 10 L of dimethylformamide (DMF) and then 0.9 mol of potassium tert-butoxide was added thereto with stirring. It was warmed to 55 °C with stirring for 30 minutes. 0.9 mol of 2-methyl-3-nitrobenzyl chloride was slowly added and reacted while mixing for an additional 2 hours. After the reaction was completed, 4 L of purified water was added and dried in a rotary evaporator (Rotavapor® R-220, BUCHI) while heating to 60°C. 14 L of dimethyl chloride was added to dissolve the dried product and 7L of distilled water was added for layer separation. After taking the supernatant, 0.7 kg of each of magnesium sulfate and activated carbon were added thereto and stirred for 1 hour, filtered through Celite, and dried using a rotary evaporator (yield: 60%).

After dissolving about 2 kg of the resulting dried product in ethanol, 100 g of iron chloride hexahydrate, 600 g of activated carbon, and 10 kg of hydrazine monohydrate were added to cool the reaction solution. Thereafter, it was filtered to obtain white precipitates, which were dried overnight at 40° C in a vacuum oven to produce 1-(3-amino-2-methylbenzyl)-4-(2-thiophen-2-yl-ethoxy)-1H-pyridin-2-one. The yield was 85%.

In order to remove the related substances generated in the synthesis process, purification may be performed as needed. Purification is carried out as follows: 2 kg of the synthesized raw material is dissolved in 30 L of dichloromethane and then purified water is added for layer separation. After taking the organic layer, 0.7 kg of sodium sulfate is added and additionally 0.7 kg of activated carbon is added thereto, and stirred for 1 hour, filtered and concentrated under reduced pressure to remove dichloromethane. Further, 10 L of ethyl acetate was added to dissolve, concentrated, and recrystallized by adding 20 L of hexane, and then dried at 40 °C. The purification operation can be repeated as needed.

Preparation Example 2: Preparation of salts of the compound of formula 1

The compound of formula 1 has very low solubility in distilled water of 2 μg/mL, which causes a decrease in bioavailability due to low solubility after administration to the body. In addition, the method for preparing polymer dispersion particles by spray drying that can be used to prepare a pharmaceutical composition is not only very complicated, but also has a disadvantage of relatively large loss of raw materials in the manufacturing process. In order to improve these problems, salts of the compound of formula 1 were prepared.

6 g of the compound of formula 1 (M.W. 340.45) according to Preparation Example 1 was added to 100 mL of a solvent dichloromethane, followed by stirring at room temperature for 30 minutes to completely dissolve it (0.176M). When an acidic substance was a liquid, it was used as it was, and when an acid substance was a solid, it was used with dissolved in water. The acidic substance was slowly added, while stirring the solution of the compound of formula 1 dissolved in the solvent at a speed of 100 rpm. The type of the acidic substance and properties according to the addition of the acid substance are shown in Table 1.

[Table 1]

| Acid substance | Addition of acid substance | After drying | Water solubility (μg/mL) |
|---|---|---|---|
| | Properties | | |
| Citric acid | Phase separation (Oil droplets) | Viscous liquid | 383 (153 times) |
| Phosphoric acid | Viscous liquid (Phase separation) | Viscous liquid | 1130 (452 times) |
| Sulfuric acid | Precipitation and browning | Brown solid | 838 (335 times) |
| Hydrochloric acid | Precipitation | Solid | 927 (370 times) |
| Fumaric acid | Liquid | Sponge-like viscous liquid | 265 (106 times) |
| Ascorbic acid | Liquid | Sponge-like viscous liquid | 100 (40 times) |
| Tartaric acid | Phase separation (Oil droplets) | Viscous liquid | 392 (157 times) |
| Acetic acid | Liquid | Sponge-like viscous liquid | 45 (18 times) |
| EDTA | Liquid | Sponge-like viscous liquid | 48 (19 times) |

From the results of the formation of salt using various types of acidic substances as shown in Table 1, addition of hydrochloric acid and sulfuric acid generate precipitates immediately. However, in the case of sulfuric acid, the color of the precipitates gradually turned brown over time. In addition, other acidic substances maintained their phase separation state or liquid forms, as shown in the table. When precipitates were generated, the precipitates were filtered, diluted with dichloromethane, and filtered again to remove any acidic substances that may remain. After repeating these processes twice, the obtained precipitates were dried for 12 hours in a vacuum oven preheated to 40 °C. The yield was 95 to 98%, and the dried product was pulverized to measure the solubility. In the case of a liquid phase or a phase separation, the sample was dried using a rotary evaporator (Tokyo Rikakikai Co., Ltd/ Eyela laboratory evaporator N-1000). Each sample obtained was measured for solubility. The results are shown in Table 1. The water solubility in neutral water of phosphate increased 452 times higher than free base, and the water solubility of sulfates and hydrochlorides increased more than 330 times. In addition, the solubilities of citrates and tartrates increased more than 150 times, and the solubilities of ascorbates and fumarates increased about 40 and 100 times, respectively, and finally solubilities of acetates and EDTA salts increased about 20 times. With comprehensively considering the ease of manufacture, properties after drying and the solubility, hydrochlorides can be applied to the salt preparation of the compound of formula 1.

Example 1: Preparation of capsules

The compound of formula 1 has very low solubility in water. It is known that the lower the solubility, the lower the bioavailability. To overcome this problem, a finished form was prepared by preparing polymer based solid dispersion particles and filling them into a capsule. More specifically, first, about 80 g of 1-(3-amino-2-methylbenzyl)-4-(2-thiophen-2-yl-ethoxy)-1H-pyridin-2-one, a salt thereof or a mixture thereof was dissolved in 1.6 L of dichloromethane, and then 80 g of a hydrophilic polymer, polyvinyl pyrrolidone and 3.26 g of a solubilizing agent, Tween 80 were added, followed by stirring at room temperature for 30 minutes to completely dissolve them. A spray drying process (GEA/Niro SDMICRO™) was carried out to form particles. The spray drying process was carried out as shown in Table 2 below and particles were collected, and then dried overnight using a vacuum oven preheated to 40 °C to remove dichloromethane, which may remain. The prepared powders were filled into hard gelatin capsules to prepare capsules.

[Table 2]

| **set up parameters** | | | | | | |
|---|---|---|---|---|---|---|
| **Inlet Temperature [°C]** | **Outlet Temperature [°C]** | **Δp Air Flow [mbar]** | **Atomising Pressure [bar]** | **Atomising flow [%]** | **P withing drying chamber [cm/$H_2O$]** | **Spray rate [g/min]** |
| 94-97 | 50-55 | (80 kg/h) | 0.7 | 35-55 | 0-60 | NMT 105 |

Example 2: Preparation of tablets containing salts.

A tablet containing the salt of the compound of formula 1 according to Preparation Example 2 was prepared. First, on a per tablet basis, to a mixture of 42.4% (w/w) of hydrochloride of the compound of formula 1, 27.1% (w/w) of microcrystalline cellulose, 20.3% (w/w) of D-mannitol, 3.4% (w/w) of croscarmellose sodium and 1.4% (w/w) of Aerosil (Tomita Pharmaceuticals (Japan), Florite®), 60 μL of a binder solution with hydroxypropylcellulose dissolved in ethanol (10% (w /v)) was slowly added to form granules and then dried for 3 to 4 hours in a hot air dryer preheated to 60 °C. The moisture content of the dried product was set to 3% or less. The dried granules were sieved with an 18-mesh sieve. After adding 2% (w/w) of croscarmellose sodium as a disintegrant and 1.4% (w/w) of magnesium stearate as a lubricant, mixing and tableting were performed to prepare tablets.

Example 3: Preparation of tablets containing nanoparticles

In order to improve the water solubility of the compound of formula 1 according to Preparation Example 1, the particle size of the compound of formula 1 was nanosized, thereby increasing the surface area of the raw material to improve the solubility. For this purpose, 1 g of the compound of formula 1 was stirred in 10 mL of dimethyl sulfoxide (DMSO) and completely dissolved to prepare a transparent solution. After dissolving 0.5 g of Poloxamer 407 in 20 mL of myristyl alcohol at 70° C, the solution of the compound of formula 1 was slowly added thereto and emulsified by mixing for 5 minutes using a homogenizer (IKA®-Werke GmbH & Co.KG, Demark/IKA® T25 digital LR) at a speed of 15,000 rpm. The temperature of the emulsification process was 80°C. After the emulsification process, it was stored at room temperature for a certain period of time to rapidly lower the temperature, and the resulting solids were pulverized to an average particle size of about 290 nm using a tory Hills T65 three roll mill. The prepared pulverized materials were placed in a supercritical extraction system (ILSHIN Autoclave/SC-CO2 extraction system) while injecting carbon dioxide. At this time, the pressure in the reactor was maintained at about 70 atm to remove myristyl alcohol and dimethyl sulfoxide and remain only solid particles. The weight of the obtained solids was 1.48 g and the yield was 98.7%. The obtained solids were measured for particle size by a zeta potential measurement system (ELS-2000Z, Otsuka Electronices Korea Co., Ltd.). As a result, the average particle size was 318 nm, and the results are shown in Fig. 1 and Table 3.

[Table 3]

| Data | Repet. No | pH | Ave. Diameter (nm) | PD | Mean. (nm) | D(10%) (nm) | D(50%) (nm) | D(90%) (nm) |
|---|---|---|---|---|---|---|---|---|
| | 1 | NA | 318.5 | -0.069 | 304.2 | 232.6 | 290.1 | 365.5 |
| Average: | | | 318.5 | -0.069 | 304.2 | 232.6 | 290.1 | 365.5 |

To a mixture of 45.5% (w/w) of nanoparticles containing the compound of formula 1 prepared as described above as a main component, 22.7% (w/w) of microcrystalline cellulose, 22.7% (w/w) of D-mannitol, 3.0% (w/w) of croscarmellose sodium and 1.4% (w/w) of Aerosil (Tomita Pharmaceuticals (Japan), Florite®), 60 μL of a binder solution with hydroxypropylmethyl cellulose (HPMC) dissolved in ethanol (10% (w /v)) was slowly added to form granules and then dried for 3 to 4 hours in a hot air dryer preheated to 60 °C. The moisture content of the dried product was set to 3% or less. The dried granules were sieved with an 18-mesh sieve. After adding 1.8% (w/w) of croscarmellose sodium as a disintegrant and 1.2% (w/w) of magnesium stearate as a lubricant, mixing and tableting were performed to prepare tablets.

Examples 4 to 8: Preparation of tablets

In order to solve the decrease in productivity and economic efficiency that may occur due to loss of raw materials in salt introduction, polymer dispersion particles and nanosized particles, the compound of formula 1 as a raw material was pulverized with a jet mill (Komachine, Micro Jet Mill) and prepared by varying particle size ($D_{90}$) value of the raw material as shown in Table 4. To the compound of formula 1 with different particle sizes, 24.0% (w/w) of microcrystalline cellulose (MCC), 21.7% (w/w) of mannitol (D-manitol), and 0.8% (w/w) of croscarmellose sodium were added which had sieved to remove lumps which may be included, and then they were mixed uniformly. After mixing, 150 μL of a binder solution with hydroxypropyl cellulose dissolved in ethanol (6% (w/v)) was added to form granules and dried until the moisture content became 2% or less. After sieving, on a per tablet basis, 0.8% (w/w) of magnesium stearate was added as a lubricant, mixed and subjected to tableting to prepare tablets.

[Table 4]

| | $D_{90}$(μm) |
|---|---|
| Example 4 | 10.1 |
| Example 5 | 25.6 |
| Example 6 | 60.1 |
| Example 7 | 124.3 |
| Example 8 | 253.1 |

Experimental Example 1: Evaluation of inhibition against MRSA strains

In order to verify the inhibitory effect of the compound of formula 1 on antibiotic-resistant strains, drug susceptibility was evaluated by treating the compound of formula 1 with *Staphylococcus aureus* phenotype isolated from each patient. In an in vitro test for antibiotic development, the most important result can be $MIC_{90}$ (minimum inhibitory concentration required to inhibit the growth of 90% of the total bacterial population). Table 5 shows the results of $MIC_{90}$ tests with representative drugs which are currently on the market as a control drug, for about 100 methicillin-susceptible strains isolated in the laboratory of Dr. Peter C. Appelbaum at Hershey Hospital, who is recognized for its authority in the field of anti-infection worldwide, and about 100 MRSA strains which is currently socially problematic.

[Table 5]

| Drug | Methicillin-susceptible (μg/mL) n=103) | | | Methicillin-resistant (μg/mL), n=100) | | |
|---|---|---|---|---|---|---|
| | Range | $MIC_{50}$ | $MIC_{90}$ | Range | $MIC_{50}$ | |
| CG400549 | 0.06 - 1.0 | 0.25 | 0.25 | 0.06 - 1. 0 | 0.25 | 0.25 |
| Vancomycin | 1.0 - 2.0 | 1.0 | 2.0 | 1.0 - >64.0 | 1.0 | 2.0 |
| Teicoplanin | 0.125 - 8.0 | 1.0 | 2.0 | 0.25 - >64.0 | 1.0 | 2.0 |
| Linezolid | 0.25 - 2.0 | 1.0 | 2.0 | 0.25 - 2.0 | 1.0 | 2.0 |
| Quinupristin-dalfopristin | 0.25 - 2.0 | 1.0 | 2.0 | 0.25 - 2.0 | 1.0 | 2.0 |
| Daptomycin | 0.25 - 2.0 | 1.0 | 1.0 | 0.25 - 4.0 | 0.5 | 0.5 |
| Amoxicillin-clavulanate | 0.125 - 4.0 | 1.0 | 2.0 | 0.5 - >64.0 | >64.0 | >64.0 |
| Azithromycin | 0.25 - >64.0 | 1.0 | >64.0 | 0.5 - >64.0 | >64.0 | >64.0 |
| Levofloxacin | ≤0.06 - 32.0 | 0.25 | 4.0 | 0.125 - >32.0 | 1.0 | >32.0 |

As shown in Table 5, it is found that the $MIC_{90}$ value is 0.25 μg/mL irrespective of susceptible strains and non-susceptible strains, i.e., MRSA strains, which indicates two to tens of times superior results compared to the control drug. In particular, these strains include vancomycin-intermediate Staphylococcus aureus (VISA) strain which is resistant to vancomycin and vancomycin-resistant Staphylococcus aureus (VRSA) strain which is a super bacterium (vancomycin MIC > 64 μg/mL). From these results, it can be seen that the compound of formula 1 can be used as an effective therapeutic agent for diseases or disorders caused by bacterial infection, compared to conventional drugs such as vancomycin, teicoplanin, linezolid, amoxicillin-clavulanate, daptomycin, etc. Specifically, although not limited thereto, it may be usefully used as a therapeutic agent for bacterial infections related to diseases including urinary tract, respiratory tract, skin tissue infection, sepsis, and the like.

Experimental Example 2: Analysis of solubility of the compound of formula 1

Prior to preparing the salt of the compound of formula 1, the compound of formula 1 was analyzed to determine the solubility in various solvents. First, the compound of formula 1 was supersaturated in a pharmaceutically acceptable solvent as shown in Table 5 and stirred in a dark room at room temperature for 12 hours. It was first centrifuged to take a supernatant, and then again filtered with a 0.25 μm PVDF filter to remove insoluble materials remaining in the solution. The filtrate was diluted with methanol and then subjected to HPLC analysis to quantify the solubility. The results are shown in Table 6. In addition, HPLC analysis conditions are as follows.

20 μL of each of the test solution and the standard solution were tested according to a liquid chromatography method (HPLC) of general test methods of the Korean Pharmacopoeia under the following conditions, and the peak area of the main component of each solution was measured.

Operating condition and calculation

[Operating condition]

Detector: UV spectrophotometer (measurement wavelength 286 nm)
Column: Aegispak C18-L (4.6 mm x 250 mm, 5 μm) column
Column temperature: 25 °C
Mobile phase: Acetonitrile: water = 3: 2
Flow rate: 1.0 mL/min

[Calculation]

$$\text{Content (\%)} = \frac{A_T}{A_S} \times \frac{D_T}{D_S} \times P$$

$A_T$ : Peak area of the main component in the test solution
$A_S$ : Peak area of the main component in the standard solution
$D_T$ : Dilution factor of the test solution
$D_S$ : Dilution factor of the standard solution
P : Purity of the main component in the standard solution (%)

[Table 6]

| Item | Solubility (mg/mL) |
|---|---|
| Water | 0.002 |
| pH 1.2 aqueous solution | 0.9 |
| Ethanol | 3.6 |
| Methanol | 6.1 |
| Glycerol | 0.3 |
| PEG 300 | 22 |
| Dichloromethane | 60 |
| DMSO | about 180 |

As shown in Table 6, it is found that the solubility increases as the pH of the compound of formula 1 decreases. For example, it had a low solubility of 2.5 μg/mL in water (neutral), but a solubility of about 0.9 mg/mL in a strong acidic condition of pH 1.2. The solubilities in ethanol, methanol, and glycerol were very low, below 10 mg/mL, and the solubilities in dichloromethane and DMSO were high, above 50 mg/mL. However, DMSO was expected to have difficulty in drying due to its low volatility from the high boiling point, so it was judged to be inappropriate for use in salt production. Therefore, in the salt production process, for example, dichloromethane that has high volatility and relatively high solubility can be used.

Experimental Example 3: Analysis of stability of capsules

The stability of the capsule of Example 1 according to the storage conditions was verified. Storage conditions

are long-term and accelerated conditions, specifically, temperature of 25±2°C and humidity of 60±5%, and temperature of 40±2°C and humidity of 75±5%, respectively. The results are shown in Table 7.

[Table 7]

| Storage condition | Storage period (month) | Dissolution test | Content | Total related substances |
|---|---|---|---|---|
| | | 85% or more in 30 minutes | 95~105% | 1% or less |
| Initial value | | 95 | 98.0 | 0.08 |
| 25±2°C/60±5% | 1 | 96 | 97.8 | 0.07 |
| | 3 | 98 | 100.4 | 0.09 |
| | 6 | 98 | 98.9 | 0.12 |
| | 9 | 99 | 101.1 | 0.15 |
| | 12 | 97 | 98.9 | 0.18 |
| 40±2°C/75±5% | 1 | 98 | 97.4 | 0.14 |
| | 3 | 99 | 99.2 | 0.07 |
| | 6 | 96 | 99.8 | 0.15 |

As shown in Table 7, it was found that the dissolution, content and related substances meet the standards by at least 12 months under long-term storage conditions and 6 months under accelerated conditions. Considering this, the shelf life of the product can be determined, for example, to be 24 months.

Experimental Example 4: Analysis of stability of tablets containing hydrochloride of the compound of formula 1

The stability of the tablet of Example 2 according to storage conditions was verified. The storage conditions were long-term and accelerated conditions, and the dissolution, content and related substances were evaluated as test items. Long-term and accelerated storage conditions are temperature of 25±2°C and humidity of 60±5%, and temperature of 40±2°C and humidity of 75±5%, respectively. The results are shown in Table 8.

[Table 8]

| Storage condition | Storage period (month) | Dissolution test | Content | Total related substances |
|---|---|---|---|---|
| | | 85% or more in 30 minutes | 95~105% | 1% or less |
| Initial value | | 89 | 100.3 | 0.04 |
| 25±2°C/60±5% | 1 | 95 | 101.2 | 0.07 |
| | 3 | 97 | 99.5 | 0.04 |
| | 6 | 95 | 100.6 | 0.05 |
| 40±2°C/75±5% | 1 | 93 | 102.1 | 0.03 |
| | 3 | 91 | 99.4 | 0.07 |
| | 6 | 96 | 100.5 | 0.11 |

As shown in Table 8, in all test items under the two conditions, the result values showed that the stability was maintained within the set range, and no significant change was observed for 6 months for each item. From this, it can be seen that the tablet containing the hydrochloride of the compound of formula 1 has excellent physical and chemical stability.

Experimental Example 5: Analysis of stability of tablets containing nanoparticles

The stability of the tablet containing nanoparticles of Example 3 according to storage conditions was verified. The storage conditions were long-term and accelerated conditions, and the dissolution, content and related substances were evaluated as test items. Long-term and accelerated storage conditions are temperature of 25±2°C and humidity

of 60±5%, and temperature of 40±2°C and humidity of 75±5%, respectively. The results are shown in Table 9.

[Table 9]

| Storage condition | Storage period (month) | Dissolution test | Content | Total related substances |
|---|---|---|---|---|
| | | 85% or more in 30 minutes | 95~105% | 1% or less |
| Initial value | | 91 | 101.1 | 0.05 |
| 25±2°C/60±5% | 1 | 94 | 102.1 | 0.06 |
| | 3 | 95 | 100.5 | 0.10 |
| | 6 | 97 | 101.3 | 0.07 |
| 40±2°C/75±5% | 1 | 92 | 101.1 | 0.06 |
| | 3 | 93 | 100.4 | 0.04 |
| | 6 | 89 | 102.5 | 0.08 |

As shown in Table 9, no significant change was observed for 6 months in all test items under both conditions. From this, it can be seen that the tablet containing the compound of formula 1 has excellent stability.

Experimental Example 6: Solubility and dissolution test

The solubility in distilled water of the compound of formula 1 and its salt, and the polymer dispersion particles and powders of nanoparticles prepared using the compound of formula 1 in Preparation Example 1, Preparation Example 2, Example 1 and Example 3 was verified, respectively. First, an excess of each substance was added to distilled water and mixed at room temperature for 12 hours. After standing for 2 hours to take the supernatant, it was filtered using a filter having 0.25 μm pores. After diluting this in methanol again, HPLC analysis was performed. The HPLC conditions are the same as the analysis conditions in the solubility test of Experimental Example 2. The results are shown in Table 10.

[Table 10]

| Items | Water solubility (μg/mL) | pH |
|---|---|---|
| Preparation Example 1 | 2.5 | 6.8 |
| Preparation Example 2 | 927 | 2.1 |
| Example 1 | 154 | 6.7 |
| Example 3 | 168 | 6.9 |

As shown in Table 10, it was found that the solubility was improved in case of salt introduction, polymer dispersion particles and nanoparticles, compared to a free base.

In addition, each of the compound of formula 1 according to Preparation Example 1, the hydrochloride salt of the compound of formula 1 according to Preparation Example 2, the polymer dispersion particle powders according to Example 1, and the nanoparticle powders according to Example 3 as mentioned above was filled into a gelatin hard capsule. Then, a dissolution test was conducted in distilled water by the dissolution test method 2 (paddle method) of general test methods of the Korean Pharmacopoeia. The results are shown in Fig. 2. The samples obtained during the experiment were analyzed by HPLC to quantify the drug content for each time period. HPLC conditions are as follows.

[Dissolution test conditions]

- Test method: Korean Pharmacopoeia dissolution test method 2 (paddle method)
- Test solution: distilled water
- Test temperature: 37±0.5°C
- Rotation speed: 50 rpm

According to the liquid chromatography method (HPLC) of the general test method of the Korean Pharmacopoeia under the following conditions, peak areas of the main component of each solution were measured.

Operating condition and calculation

[Operating condition]

The operating condition and calculation equation are the same as those of content test used in the solubility test of Experimental Example 2. As shown in Fig. 2, it is found that the dissolution pattern in water was remarkably improved when the techniques of Preparation Example 2 (salts), Example 1 (polymer dispersion particles) and Example 2 (nanoparticles) were applied, compared to Preparation Example 1.

Experimental Example 7: Analysis of dissolution pattern according to particle size

Prior to the dissolution test of the tablets according to Examples 4 to 8, a test for uniformity of dosage forms was performed. In the test for uniformity of dosage forms, 10 samples in each Example was prepared and the content per tablet for each sample was quantified. The acceptance value of uniformity of dosage forms was determined according to the content uniformity test of the general test method of the Korean Pharmacopoeia. In general, the acceptance value is inversely proportional to the uniformity of dosage form. The acceptance values of Examples 4 to 8 are shown in Table 11.

[Table 11]

| Example | Uniformity of dosage forms (AV: %) |
|---|---|
| Example 4 | 2.5 |
| Example 5 | 2.8 |
| Example 6 | 4.8 |
| Example 7 | 5.7 |
| Example 8 | 6.8 |

As shown in Table 11, the acceptance value tends to increase as the particle size of the raw material used increases. That is, as the particle size increases, the uniformity of dosage forms decreases. In particular, when the particle size distribution ($D_{90}$) is about 50 $\mu$m, the acceptance value for uniformity of dosage forms increases rapidly, which is considered to represent a decrease in uniformity of dosage forms.

In order to determine the difference in dissolution rate according to the particle size of raw materials, an experiment was conducted according to the dissolution test method 2 (paddle method) of general test methods of the Korean Pharmacopoeia. The experiment was conducted using 900 mL of distilled water and pH 1.2 aqueous solution as a dissolution medium at a rotation speed of 50 rpm and a temperature of 37±0.5°C. The results of using distilled water as a dissolution medium are shown in Fig 3, and the results of using the pH 1.2 aqueous solution as a dissolution medium are shown in Fig 4.

The dissolution medium was taken at regular time intervals, filtered through a 0.45 $\mu$m filter, and then subjected to HPLC analysis to evaluate the degree of dissolution. HPLC analysis conditions are the same as the dissolution test method used in Experimental Example 6. As shown in Figs. 3 and 4, as the particle size of the raw material decreases, the dissolution rates in distilled water and a pH 1.2 dissolution medium are improved. In particular, Example 4 showed a similar dissolution rate in distilled water to those in case of applying the techniques of Preparation Example 2 (salt introduction), Example 1 (polymer dispersion particles) and Example 2 (nanoparticles).

In addition, the difference in dissolution rates for each sample also gradually decreased. Specifically, in the case of Examples 4 and 5, the deviation of the dissolution test is within 1 to 3%, whereas in the case of Examples 6 to 8, it shows 3 to 10%. From the above, it is found as the particle size is finer, the uniformity of dosage unit is improved, the dissolution rate is improved, and the dissolution deviation decreases. Therefore, it is expected to show a faster and more consistent body absorption pattern when administered orally, thereby expecting to guarantee a rapid and constant therapeutic effect.

Experimental Example 8: Analysis of stability of the tablet according to Example 4

After storing the tablet according to Example 4 in a plastic bottle, it was observed whether a significant change in the physical and chemical properties of the product under long-term conditions of 25±2°C and 60±5%RH and accelerated conditions of 40±2°C and 75±5%RH. The results are shown in Table 12.

[Table 12]

| Storage condition | Storage period (month) | Dissolution test | Content | Total related substances |
|---|---|---|---|---|
| | | 85% or more in 30 minutes | 95-105% | 1% or less |
| Initial value | | 89 | 100.3 | N.A. |
| 25±2°C/60±5% | 1 | 87 | 100.1 | 0.04 |
| | 3 | 91 | 99.7 | 0.06 |
| | 6 | 92 | 100.5 | 0.05 |
| 40±2°C/75±5% | 1 | 89 | 99.8 | 0.08 |
| | 3 | 91 | 101.2 | 0.10 |
| | 6 | 89 | 99.5 | 0.09 |

As shown in Table 12, no significant change was observed for 6 months in all test items under long-term and accelerated conditions. From this, it can be seen that the tablet has stability. In Examples 5 to 8, it is expected that they will be physically and chemically very stable under the same conditions although the results are not shown.

Experimental Example 9: Pharmacokinetic and pharmacodynamic analysis in mouse model

For the compound of formula 1, pharmacokinetic/pharmacodynamic experiments were conducted using a mouse infection model. To this end, experiments were conducted with Staphylococcus aureus ATCC 29213 (MSSA, standard strain) and 13B-382 (MRSA, clinical strain). As a medium, Mueller-Hinton broth or Cation-adjusted Mueller-Hinton broth was used. For the susceptibility test (minimum inhibitory concentration (MIC)), the compound of formula 1 was used.

With aseptic (Specific pathogen free, SPF) female, 6 weeks old (23~27g) ICR mouse (Orient Bio Inc, Gapyeong, Korea), the experiment was carried out in compliance with the regulations and procedures with the permission of the Ethics committees for animal experiments in accordance with the Animal Protection Act and the Laboratory Animal Act. Cyclophosphamide (Bexter, Frankfurt, Germany) was injected subcutaneously to induce reduction in neutrophils (<100/mm$^3$). Before the experiment, the test strains were incubated in Muller Hinton II broth for 24 hours at 37°C to obtain a concentration of $10^8$ CFU/mL. Then, they were diluted with physiological saline. 0.1 ml of the solution was inoculated into the thigh of the mouse (inoculation amount 1.0 x $10^5$ CFU/mL). After 2 hours, oral administration of the compound of formula 1 was started. Drugs were administered every 3, 6, 12 and 24 hours at a dose of 7.5 mg to 240 mg/kg/day.

After 24 hours of drug administration, the mouse was euthanized with carbon dioxide gas to separate its thigh, put in physiological saline, and cut finely with a homogenizer (Kinematica AG/ Polytron®). It was diluted 10 times, spread on Muller Hinton II broth, incubated at 37 °C for 24 hours. The number of viable cells was counted and recorded. The results were expressed as log10 CFU/thigh, and the measurement limit of the number of viable cells in the laboratory was 1 x $10^2$ CFU/thigh.

T/MIC was evaluated as an index to determine the effect of antibiotics in combination with the antimicrobial action and pharmacokinetic results according to the antimicrobial dosage and administration. The T/MIC value represents the percentage of a dosage interval in which the serum level exceeds the MIC. The results are shown in Fig. 5. As shown in Fig. 5, it is found that as the T/MIC value increases, the effect of eradicating bacteria increases rapidly. When the T/MIC value was approximately 20% or higher, more than 99.9% of bacteria were eradicated. In addition, it is found that when the values of $AUC_{0-24h}$/MIC and $C_{max}$/MIC are increased, the effect of eradicating bacteria such as MRSA strains also increases rapidly.

As shown in Fig. 5, it can be seen that the MIC values for Staphylococcus aureus ATCC 29213 and 13B-382 of the compound of formula 1 are 0.25 μg/mL, regardless of the strain. This value is lower than those of Oxacillin (0.25 and 16 μg/mL) and Vancomycin (0.5 and 1 μg/mL).

Experimental Example 10: Evaluation of pharmacokinetics in beagle dog model

For the formulations according to Examples 4 and 8, pharmacokinetic evaluation for beagle dogs (n = 5) was performed. Prior to the pharmacokinetic test, the beagle dog was fed at 30 g/head/day for about 30 minutes before administration of the test substance, and the test substance was administered under non-fasting. The dose was approximately 5 mg/kg, and blood samples were obtained from the jugular vein at 9 time points of 0 minutes, 10 minutes, 30 minutes, 1 h, 2 h, 4 h, 6 h, 8 h and 24 h per individual. After treatment of the blood samples, the concentration of the drug in the plasma was determined to obtain a pharmacokinetic profile as shown in Fig. 6.

The pharmacokinetic profile was greatly influenced by the particle size of the raw materials used in tablet manufacturing. As shown in Fig. 6, $AUC_{0-24h}$ and $C_{max}$ values tend to increase as the particle size decreases. In addition, the time during which antibiotic concentrations in plasma are above the MIC (T>MIC) also tended to increase as the particle size is smaller. Based on the results according to Experimental Example 9, it can be seen that changes in $AUC_{0-24h}$, the $C_{max}$ value, and the time during which antibiotic concentrations in plasma are above the MIC have a great influence on the antibacterial effect of antibiotics. As the three indices of the $AUC_{0-24h}$, $C_{max}$ value and MIC value increase, the antimicrobial activity increases significantly.

As described above, it can be seen that the present invention can be effectively applied to the treatment of multidrug resistant bacterial infections.

The above descriptions are merely illustrative of the technical idea of the present invention, and those of ordinary skill in the technical field to which the present invention pertains can make various modifications and variations without departing from the essential characteristics of the present invention. In addition, the embodiments disclosed in the present invention are not intended to limit the technical idea of the present invention, but to explain the technical idea, and the scope of the technical idea of the present invention is not limited by these embodiments. The scope of protection of the present invention should be interpreted by the appended claims, and all technical ideas within the scope equivalent thereto should be interpreted as being included in the scope of the present invention.

## Claims

1. A pharmaceutical composition for oral administration comprising 1-(3-amino-2-methylbenzyl)-4-(2-thiophen-2-yl-ethoxy)-1H-pyridin-2-one or a salt thereof.

2. The pharmaceutical composition for oral administration according to claim 1, wherein the 1-(3-amino-2-methylbenzyl)-4-(2-thiophen-2-yl-ethoxy)-1H-pyridin-2-one or a salt thereof has a particle size distribution $D_{90}$ of 0.1 to 500 $\mu$m.

3. The pharmaceutical composition for oral administration according to claim 1, wherein the composition is provided in the form of a tablet or capsule.

4. The pharmaceutical composition for oral administration according to claim 1, wherein the 1-(3-amino-2-methylbenzyl)-4-(2-thiophen-2-yl-ethoxy)-1H-pyridin-2-one or a salt thereof or both of them is present in an amount of 10 to 60% by weight based on the total weight of the composition.

5. A method for preparing a pharmaceutical composition for oral administration, comprising:

   adding 4-benzyloxy-1H-pyridone, 2-methyl-3-nitro-benzylchloride and potassium tert-butoxide to dimethylformamide, and mixing them to react with heating;
   adding purified water and drying with heating to obtain a dried product;
   dissolving the dried product in an organic solvent and adding purified water for layer separation;
   recovering the organic layer to filter and concentrate it to obtain concentrates;
   re-concentrating the concentrates and adding hexane to obtain precipitates;
   dissolving the resulting precipitates, and cooling, filtering and drying them to obtain a dried product; and
   dissolving the resulting dried product in an organic solvent, adding iron chloride hexahydrate, activated carbon and hydrazine monohydrate thereto, cooling and filtrating them to obtain resulting precipitates and then drying and pulverizing it.

6. The method for preparing a pharmaceutical composition for oral administration according to claim 5, wherein the method further comprises adding an acidic substance.

7. The method for preparing a pharmaceutical composition for oral administration according to claim 6, wherein the acidic substance includes hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, oxalic acid, fumaric acid, malonic acid, maleic acid, methanesulfonic acid, benzenesulfonic acid, p-toluenesulfonic acid, naphthalenesulfonic acid, EDTA, and combinations thereof.

**8.** The method for preparing a pharmaceutical composition for oral administration according to claim 5, wherein the precipitates are formulated into a tablet containing nanoparticles or a capsule containing solid dispersion particles.

**9.** The pharmaceutical composition for oral administration according to claim 1, wherein the composition is used for the treatment of bacterial infections.

[Fig. 1]

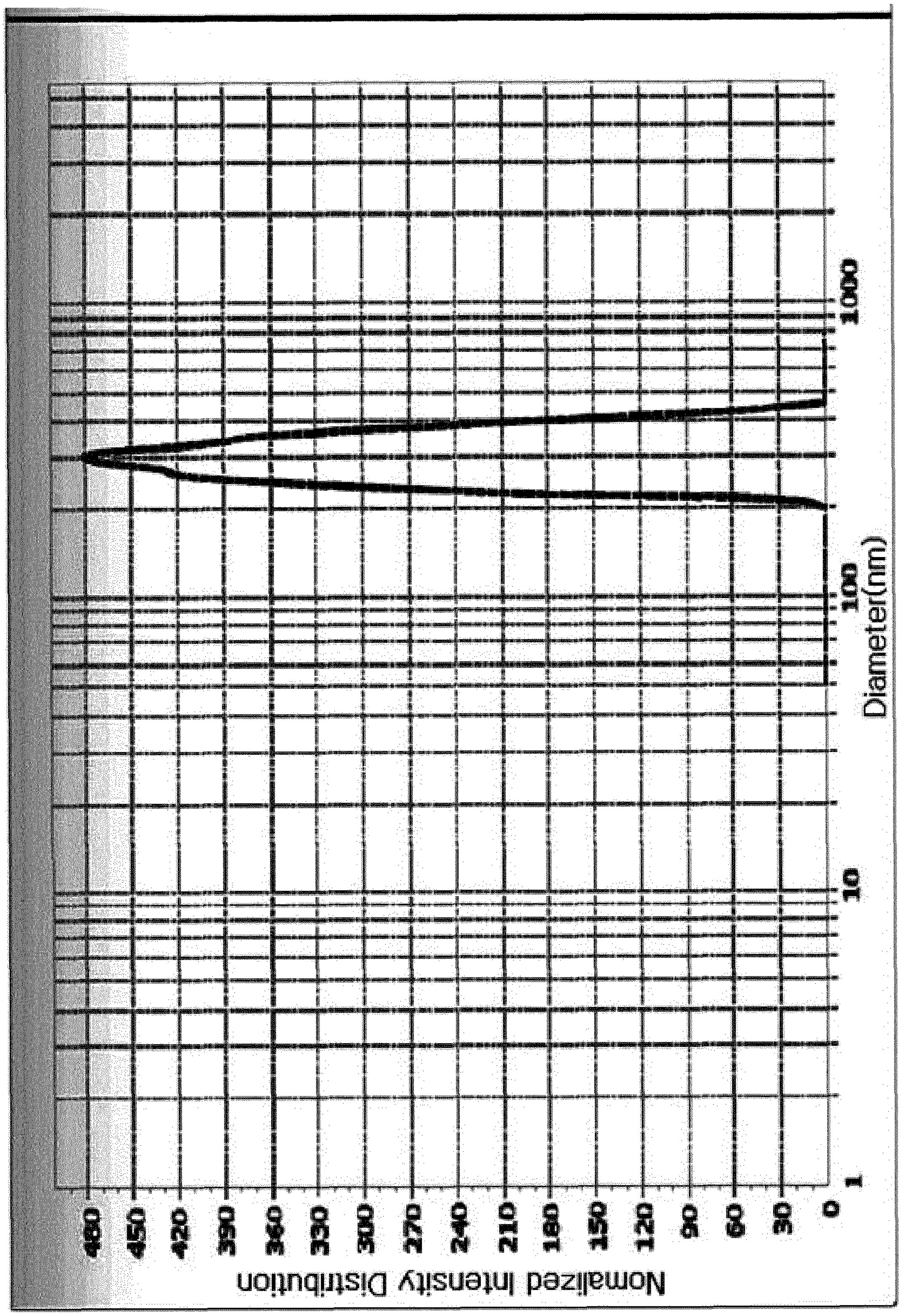
Nomalized Intensity Distribution
480
450
420
390
360
330
300
270
240
210
180
150
120
90
60
30
0
Diameter(nm)
1
10
100
1000

[Fig. 2]

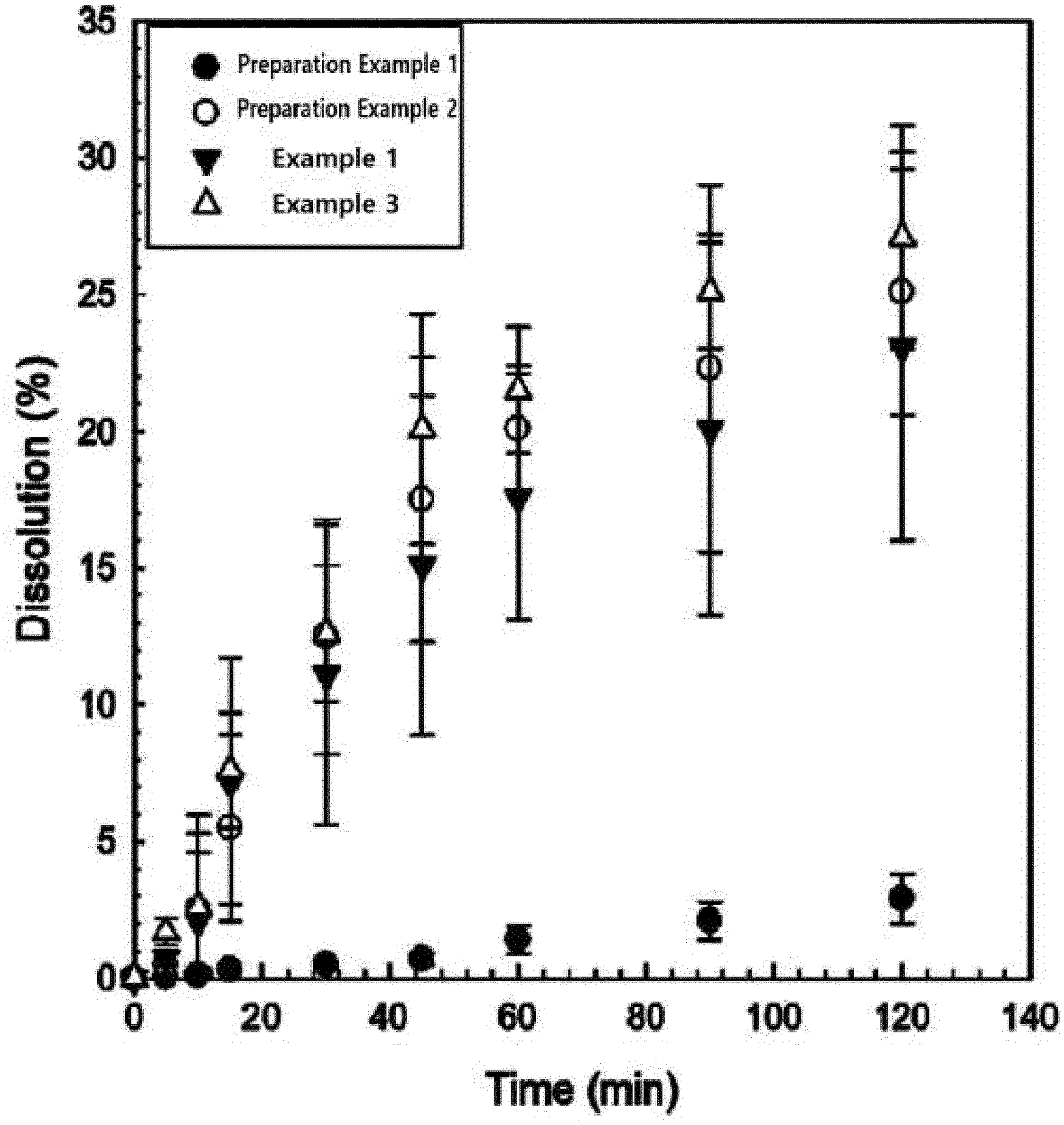
Preparation Example 1
Preparation Example 2
Example 1
Example 3
Dissolution (%)
Time (min)
0
5
10
15
20
25
30
35
0
20
40
60
80
100
120
140

[Fig. 3]

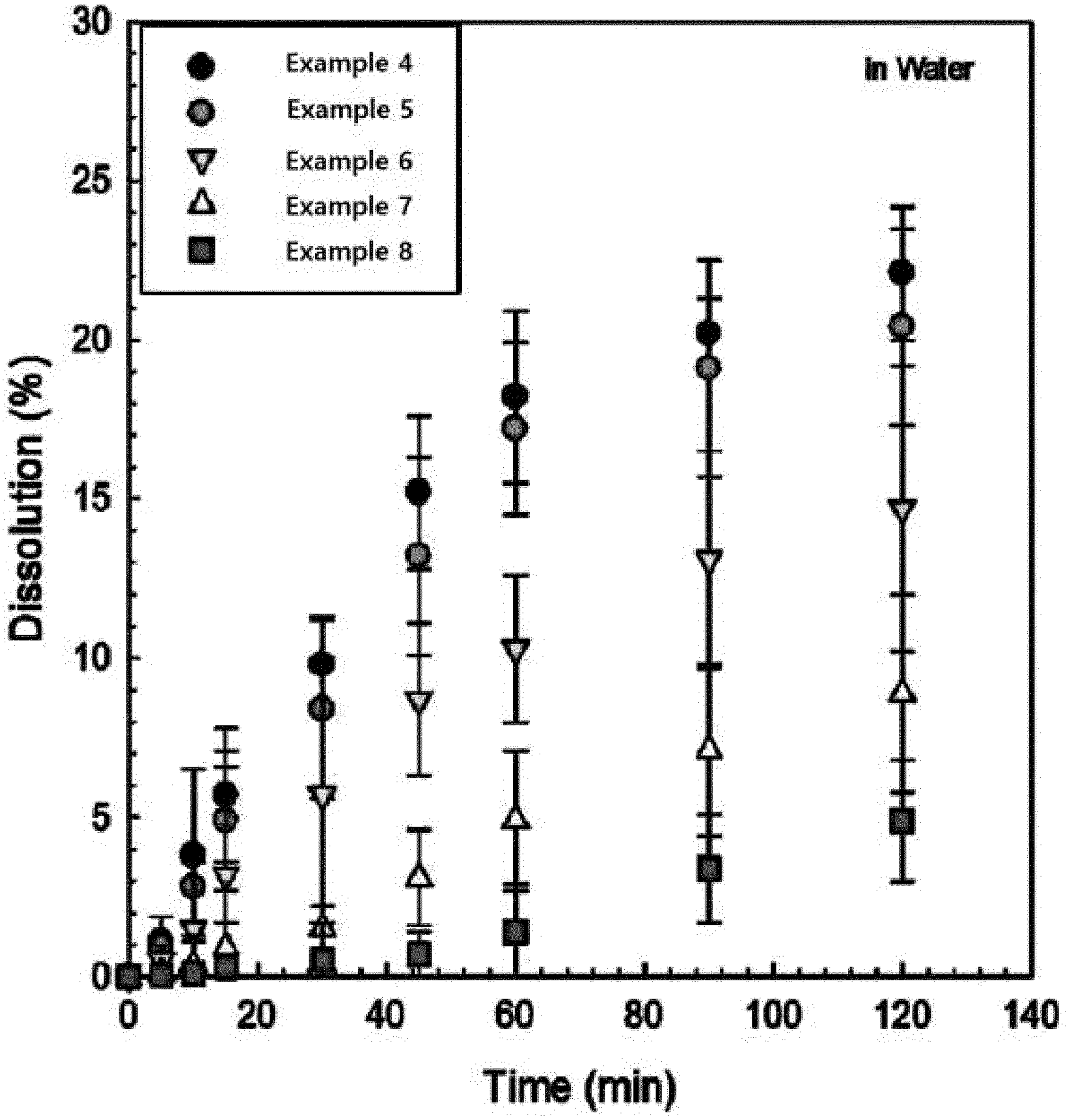
Example 4
Example 5
Example 6
Example 7
Example 8
In Water
Dissolution (%)
Time (min)
0
5
10
15
20
25
30
0
20
40
60
80
100
120
140

[Fig. 4]

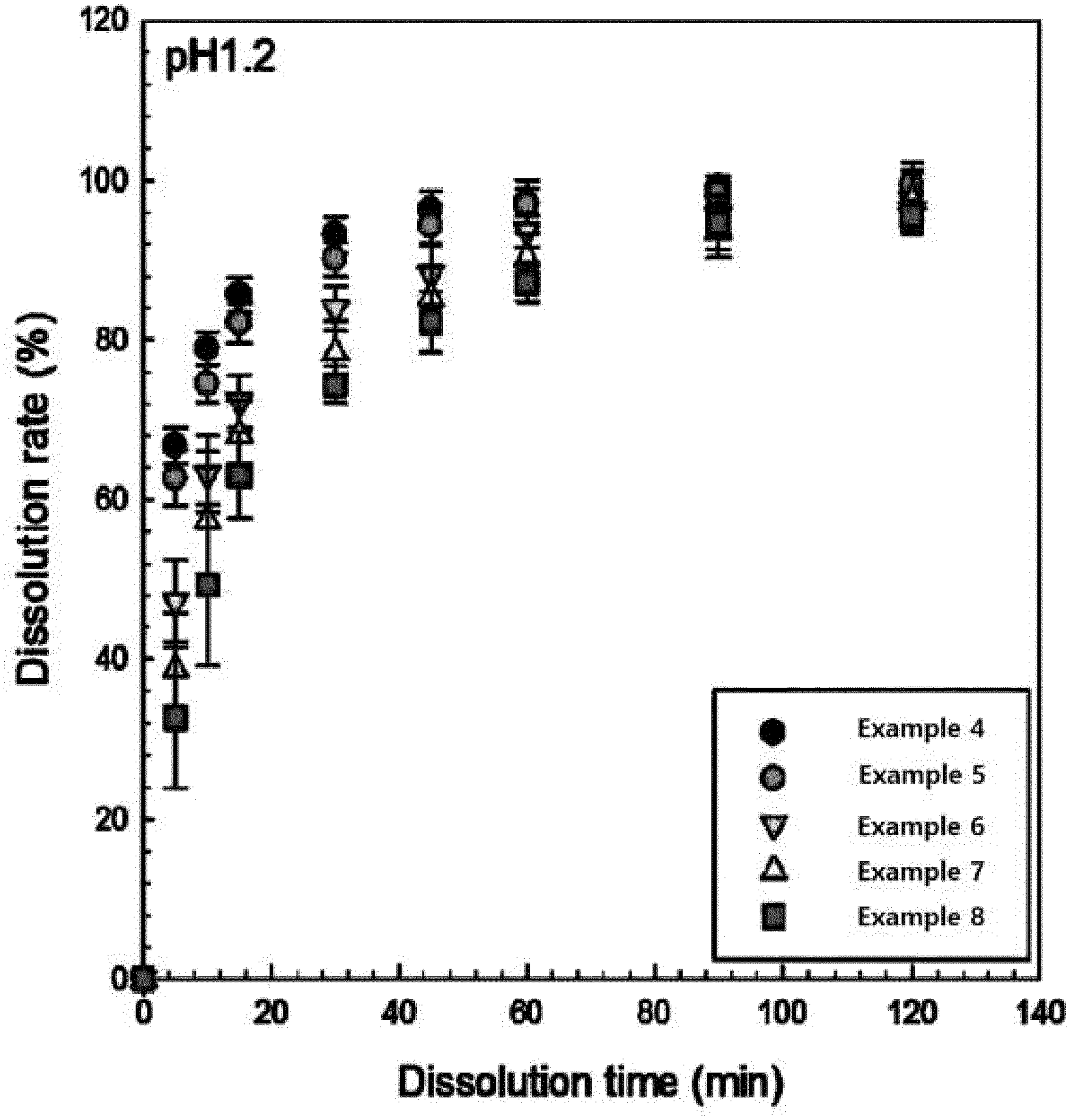
pH1.2
Dissolution rate (%)
Dissolution time (min)
0
20
40
60
80
100
120
140
Example 4
Example 5
Example 6
Example 7
Example 8

[Fig. 5]

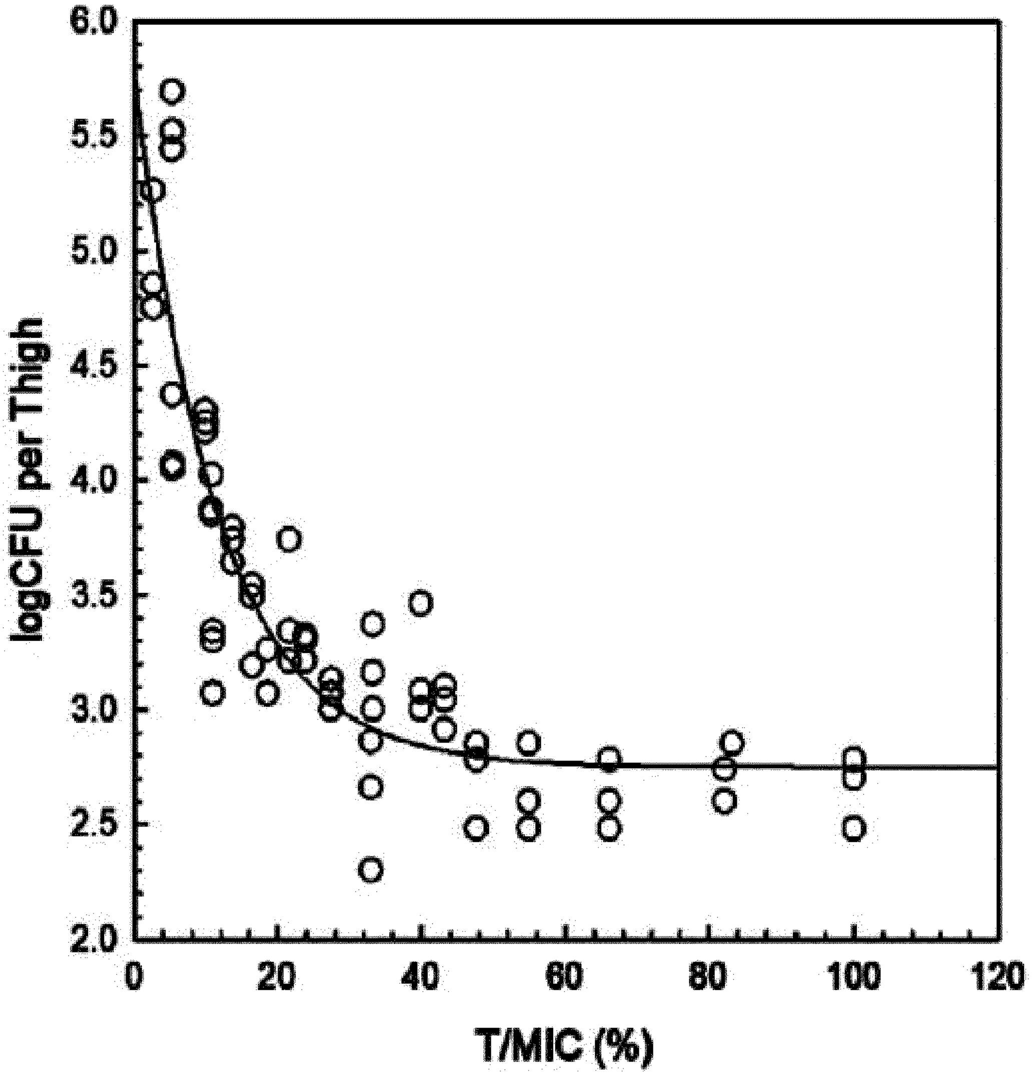
6.0
5.5
5.0
4.5
4.0
3.5
3.0
2.5
2.0
logCFU per Thigh
0
20
40
60
80
100
120
T/MIC (%)

[Fig. 6]

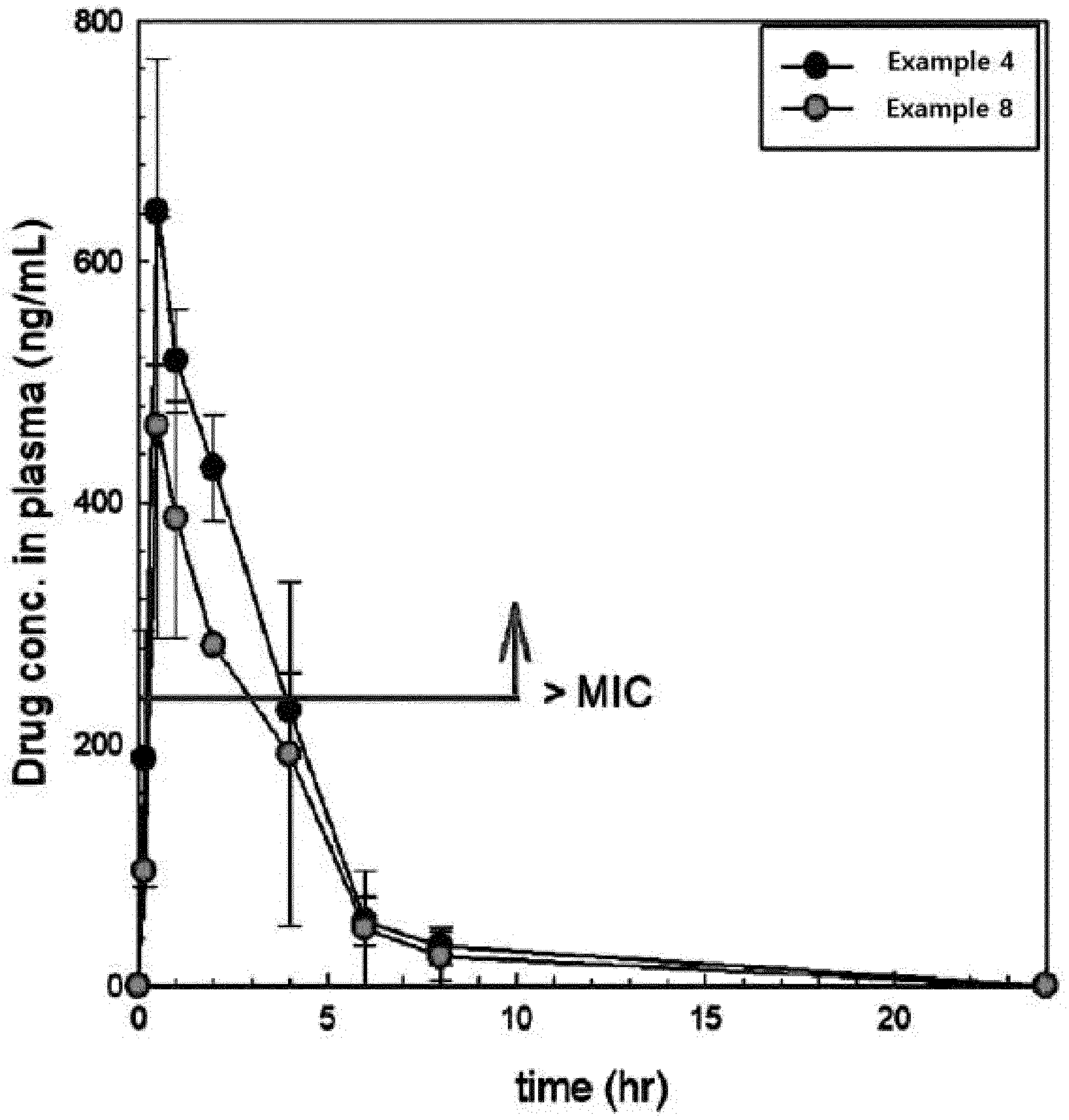
Example 4
Example 8
Drug conc. in plasma (ng/mL)
800
600
400
200
0
> MIC
0
5
10
15
20
time (hr)

**INTERNATIONAL SEARCH REPORT**

International application No.
PCT/KR2019/006432

A. CLASSIFICATION OF SUBJECT MATTER

*A61K 31/4436(2006.01)i, A61K 9/20(2006.01)i, A61K 9/48(2006.01)i, A61P 31/04(2006.01)i*

According to International Patent Classification (IPC) or to both national classification and IPC

B. FIELDS SEARCHED

Minimum documentation searched (classification system followed by classification symbols)
A61K 31/4436; A61K ; A61K 31/4045; A61K 31/44; C07D 209/14; C07D 209/18; C07D 471/04; A61K 9/20; A61K 9/48; A61P 31/04

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: FAB I inhibitor, oral administratation, tablet, bacteria, acid, synthesis method, potassium t-butoxide

C. DOCUMENTS CONSIDERED TO BE RELEVANT

| Category* | Citation of document, with indication, where appropriate, of the relevant passages | Relevant to claim No. |
|---|---|---|
| X | KR 10-2008-0068060 A (CRYSTALGENOMICS, INC.) 22 July 2008<br>See abstract; paragraphs [0026], [0049], [0354], [0367]-[0369], [0802]; claims 1-27. | 1-9 |
| A | KR 10-2016-0014987 A (CRYSTALGENOMICS, INC.) 12 February 2016<br>See abstract; paragraphs [0019], [0053]-[0055]; claims 1-7. | 1-9 |
| A | SCHIEBEL, J. et al. Rational design of broad spectrum antibacterial activity based on a clinically relevant enoyl-acyl carrier protein (ACP) reductase inhibitor. The Journal of Biological Chemistry. 2014, vol. 289, no. 23, pages 15987-16005<br>See the entire document. | 1-9 |
| A | US 6762201 B1 (MILLER, W. H. et al.) 13 July 2004<br>See the entire document. | 1-9 |
| A | WO 2004-082586 A2 (AFFINIUM PHARMACEUTICALS, INC.) 30 September 2004<br>See the entire document. | 1-9 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 AUGUST 2019 (28.08.2019) | **28 AUGUST 2019 (28.08.2019)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**
Information on patent family members

International application No.
**PCT/KR2019/006432**

| Patent document cited in search report | Publication date | Patent family member | Publication date |
|---|---|---|---|
| KR 10-2008-0068060 A | 22/07/2008 | CN 101282930 A | 08/10/2008 |
| | | CN 101282930 B | 24/10/2012 |
| | | EP 1948601 A1 | 30/07/2008 |
| | | EP 1948601 A4 | 06/04/2011 |
| | | EP 1948601 B1 | 13/04/2016 |
| | | JP 2009-511575 A | 19/03/2009 |
| | | JP 5049977 B2 | 17/10/2012 |
| | | KR 10-1502335 B1 | 16/03/2015 |
| | | KR 10-1522713 B1 | 26/05/2015 |
| | | KR 10-2014-0029550 A | 10/03/2014 |
| | | US 2007-0135465 A1 | 14/06/2007 |
| | | US 7973060 B2 | 05/07/2011 |
| | | WO 2007-043835 A1 | 19/04/2007 |
| KR 10-2016-0014987 A | 12/02/2016 | None | |
| US 6762201 B1 | 13/07/2004 | EP 1225895 A1 | 31/07/2002 |
| | | EP 1225895 B1 | 04/05/2005 |
| | | JP 2003-511415 A | 25/03/2003 |
| | | JP 4961084 B2 | 27/06/2012 |
| | | WO 01-26654 A1 | 19/04/2001 |
| WO 2004-082586 A2 | 30/09/2004 | EP 1608377 A2 | 28/12/2005 |
| | | EP 1608377 B1 | 01/10/2008 |
| | | JP 2006-523207 A | 12/10/2006 |
| | | JP 4880448 B2 | 22/02/2012 |
| | | US 2006-0142265 A1 | 29/06/2006 |
| | | US 2012-0010127 A1 | 12/01/2012 |
| | | US 7879872 B2 | 01/02/2011 |
| | | WO 2004-082586 A3 | 23/12/2004 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **PAYNE et al.** *Drug Discovery Today,* 2001, vol. 6, 537-544